# EUROPÄISCHE PATENTANMELDUNG

(11) **EP 1 431 277 A1**
(43) Veröffentlichungstag der Anmeldung: **23.06.2004**
(21) Anmeldenummer: 02028328.9
(22) Anmeldetag: 17.12.2002
(51) Int. Cl.: C07C 233/18, A01N 37/20, A01N 37/26

(54) **Amidverbindungen und ihre Verwendung zur Abwehr von Ungeziefer**

(71) Anmelder: Novartis AG, 4056 Basel (CH)
(72) Erfinder: Die Erfindernennung liegt noch nicht vor
(74) Vertreter: Gros, Florent

(57) **Zusammenfassung**

Es werden neue Verbindungen Formel ( I ) 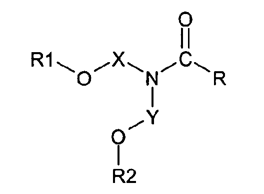 beschrieben, worin R für unverzweigtes oder verzweigtes C₁-C₁₅ Alkyl, vorzugsweise verzweigtes C₁-C₉ Alkyl steht, welches unsubstituiert oder durch Halogen, Cyano oder Nitro substituiert ist; R1 und R2 für unverzweigtes oder verzweigtes C₁-C₁₂ Alkyl, vorzugsweise C₁-C₆ Alkyl stehen, welches unsubstituiert oder durch Halogen, Cyano oder Nitro substituiert ist; und X und Y unabhängig voneinander eine geradkettige oder verzweigte Alkylenbrücke mit 1 bis 20 Kohlenstoffatomen, vorzugsweise eine Alkylenbrücke mit 1 bis 3 Kohlenstoffatomen bilden, welche unsubstituiert oder durch Halogen, Cyano oder Nitro substituiert ist. Ferner wird ein im wesentlichen ein nicht-therapeutisches Verfahren zum Fernhalten von Ungeziefer beschrieben, das auf dem Einsatz dieser Verbindungen beruht, sowie ungeziefer-abweisende Mittel, die diese Verbindungen als Wirkstoff enthalten und letztlich die Verwendung dieser Verbindungen zur Herstellung von Ungeziefer-abwehrenden Mitteln und deren Verwendung zur Abwehr von Ungeziefer an tieren, Menschen und Gegenständen.

## Beschreibung

Die vorliegende Erfindung betrifft die unter Formel (I) genannten neuen Verbindungen und ein im wesentlichen ein nicht-therapeutisches Verfahren zum Fernhalten von Ungeziefer, das auf dem Einsatz dieser Verbindungen der Formel ( I ) beruht. Ferner betrifft sie entsprechende ungeziefer-abweisende Mittel, die diese Substanzen als Wirkstoff enthalten, Verbindungen der Formel ( I ) zur Herstellung von Ungeziefer-abwehrenden Mitteln und die Verwendung von Verbindungen der Formel ( I ) zur Abwehr von Ungeziefer.

Bei den neuen Substanzen handelt es sich um Verbindungen der Formel ( I ) 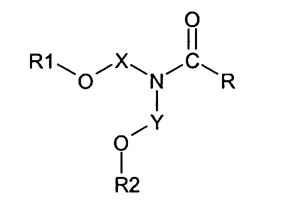 worin
R für unverzweigtes oder verzweigtes C₁-C₁₅ Alkyl, vorzugsweise verzweigtes C₁-C₉ Alkyl steht, welches unsubstituiert oder durch Halogen, Cyano oder Nitro substituiert ist;
R1 und R2 für unverzweigtes oder verzweigtes C₁-C₁₂ Alkyl, vorzugsweise C₁-C₆ Alkyl stehen, welches unsubstituiert oder durch Halogen, Cyano oder Nitro substituiert ist; und
X und Y unabhängig voneinander eine geradkettige oder verzweigte Alkylenbrücke mit 1 bis 20 Kohlenstoffatomen, vorzugsweise eine Alkylenbrücke mit 1 bis 3 Kohlenstoffatomen bilden, welche unsubstituiert oder durch Halogen, Cyano oder Nitro substituiert ist.

Eine bevorzugte Untergruppe bilden solche Verbindungen im Umfang der Formel (I), worin R für CH(C₁-C₄ alkyl)₂ steht wobei die beiden verschieden oder vorzugsweise gleich lang und uverzweigt oder vorzugsweise unverzweigt sind.

Eine weitere bevorzugte Gruppe im Rahmen der Verbindungen der Formel ( I ) besteht aus denjenigen Verbindungen bei denen X und Y unabhängig voneinander für Methylen oder Ethylen stehen.

Noch eine bevorzugte Gruppe im Rahmen der Verbindungen der Formel ( I ) besteht aus denjenigen Verbindungen bei denen R1 und R2 unabhängig voneinander für Methylen oder Ethylen stehen.

Es wurde überraschenderweise gefunden, dass die Verbindungen Formel ( I ) sich ausgezeichnet dazu eignen, Ungeziefer fernzuhalten. Durch die erfindungsgemässe Anwendung der obigen Verbindungen lässt sich unterschiedlichstes Ungeziefer von Menschen, Tieren, Sachen oder bestimmten Orten fernhalten, wobei sich zahlreiche Verbindungen im Umfang der Formel (I) durch eine besonders lange Wirkungdauer auszeichnen.

Die in den Substituentendefinitionen vorkommenden Alkylgruppen können je nach Zahl der Kohlenstoffatome geradkettig oder vorzugsweise verzweigt sein und stehen beispielsweise für Methyl, Ethyl, Propyl, Butyl, Pentyl, Hexyl, Heptyl, Octyl, Nonyl, Decyl, Undecyl, Dodecyl, Tetradecyl, Hexadecyl, Octadecyl oder Eicosyl sowie vorzugsweise deren verzweigte Isomeren, z.B. Isopropyl, Isobutyl, sek.-Butyl, tert.-Butyl, Isopentyl, Neopentyl oder Isohexyl, etc. Bevorzugt im Rahmen der vorliegenden Erfindung sind Verbindungen der Formel (I) worin R für -CH(CH₃)CH₃, -CH(CH₃)CH₂CH₃, -CH(CH₃)(CH₂)₂CH₃, -CH(CH₃)(CH₂)₃CH₃, -CH(CH₃)(CH₂)₄CH₃, -CH(CH₃)(CH₂)₅CH₃, -CH(CH₃)(CH₂)₆CH₃, -CH(CH₃)(CH₂)₇CH₃, -CH(CH₃)(CH₂)₈CH₃, -CH(CH₃)(CH₂)₉CH₃, -CH(CH₃)(CH₂)₁₀CH₃, -CH(CH₃)(CH₂)₁₁CH₃, -CH(CH₃)(CH₂)₁₂CH₃, -CH(CH₂CH₃)CH₂CH₃, -CH(CH₂CH₃)(CH₂)₂CH₃, -CH(CH₂CH₃)(CH₂)₃CH₃, -CH(CH₂CH₃)(CH₂)₄CH₃, -CH(CH₂CH₃)(CH₂)₅CH₃, -CH(CH₂CH₃)(CH₂)₆CH₃, -CH(CH₂CH₃)(CH₂)₇CH₃, -CH(CH₂CH₃)(CH₂)₈CH₃, -CH(CH₂CH₃)(CH₂)₉CH₃, -CH(CH₂CH₃)(CH₂)₁₀CH₃, -CH(CH₂CH₃)(CH₂)₁₁CH₃, -CH(CH₂CH₂CH₃)CH₂CH₃, CH(CH₂CH₂CH₃)(CH₂)₂CH₃, CH(CH₂CH₂CH₃)(CH₂)₃CH₃, CH(CH₂CH₂CH₃)(CH₂)₄CH₃, CH(CH₂CH₂CH₃)(CH₂)₅CH₃, CH(CH₂CH₂CH₃)(CH₂)₆CH₃, CH(CH₂CH₂CH₃)(CH₂)₇CH₃, CH(CH₂CH₂CH₃)(CH₂)₈CH₃, CH(CH₂CH₂CH₃)(CH₂)₉CH₃, CH(CH₂CH₂CH₃)(CH₂)₁₀CH₃, CH(CH₂CH₂CH₂CH₃)CH₂CH₃, CH(CH₂CH₂CH₂CH₃)(CH₂)₂CH₃, CH(CH₂CH₂CH₂CH₃)(CH₂)₃CH₃, CH(CH₂CH₂CH₂CH₃)(CH₂)₄CH₃, CH(CH₂CH₂CH₂CH₃)(CH₂)₅CH₃, CH(CH₂CH₂CH₂CH₃)(CH₂)₆CH₃, CH(CH₂CH₂CH₂CH₃)(CH₂)₇CH₃, CH(CH₂CH₂CH₂CH₃)(CH₂)₈CH₃, CH(CH₂CH₂CH₂CH₃)(CH₂)₉CH₃, CH(CH₂CH₂CH₂CH₂CH₃)CH₂CH₃, CH(CH₂CH₂CH₂CH₂CH₃)(CH₂)₂CH₃, CH(CH₂CH₂CH₂CH₂CH₃)(CH₂)₃CH₃, CH(CH₂CH₂CH₂CH₂CH₃)(CH₂)₄CH₃, CH(CH₂CH₂CH₂CH₂CH₃)(CH₂)₅CH₃, CH(CH₂CH₂CH₂CH₂CH₃)(CH₂)₆CH₃, CH(CH₂CH₂CH₂CH₂CH₃)(CH₂)₇CH₃, CH(CH₂CH₂CH₂CH₂CH₂CH₃)CH₂CH₃, CH(CH₂CH₂CH₂CH₂CH₂CH₃)(CH₂)₂CH₃, CH(CH₂CH₂CH₂CH₂CH₂CH₃)(CH₂)₃CH₃, CH(CH₂CH₂CH₂CH₂CH₂CH₃)(CH₂)₄CH₃, CH(CH₂CH₂CH₂CH₂CH₂CH₃)(CH₂)₅CH₃, CH(CH₂CH₂CH₂CH₂CH₂CH₃)(CH₂)₆CH₃, CH(CH₂CH₂CH₂CH₂CH₂CH₃)(CH₂)₇CH₃, CH(CH₂CH₂CH₂CH₂CH₂CH₂CH₃)CH₂CH₃, CH(CH₂CH₂CH₂CH₂CH₂CH₂CH₃)(CH₂)₂CH₃, CH(CH₂CH₂CH₂CH₂CH₂CH₂CH₃)(CH₂)₃CH₃, CH(CH₂CH₂CH₂CH₂CH₂CH₂CH₃)(CH₂)₄CH₃, CH(CH₂CH₂CH₂CH₂CH₂CH₂CH₃)(CH₂)₅CH₃, CH(CH₂CH₂CH₂CH₂CH₂CH₂CH₃)(CH₂)₆CH₃, CH(CH₂CH₂CH₂CH₂CH₂CH₂CH₂CH₃)CH₂CH₃, CH(CH₂CH₂CH₂CH₂CH₂CH₂CH₂CH₃)(CH₂)₂CH₃, CH(CH₂CH₂CH₂CH₂CH₂CH₂CH₂CH₃)(CH₂)₃CH₃, CH(CH₂CH₂CH₂CH₂CH₂CH₂CH₂CH₃)(CH₂)₄CH₃, und CH(CH₂CH₂CH₂CH₂CH₂CH₂CH₂CH₃)(CH₂)₅CH₃. Unter diesen am meisten bevorzugt sind solche Gruppen bei denen die CH-Gruppe symmetrisch substituiert ist. Ganz besonders bevorzugt ist CH(CH₂CH₂CH₃)(CH₂)₂CH₃.

Halogen und bedeutet in der Regel Fluor, Chlor, Brom oder Jod, vorzugsweise Fluor oder Chlor, insbesondere Chlor, wobei der entsprechende Alkylsubstituent ein oder mehrere gleiche oder verschiedene Halogenatome enthalten kann. Nichtlimitierende Beispiele halogenierter Alkylsubstituenten sind das ein- bis dreifach durch Fluor, Chlor und/oder Brom substituierte Methyl, wie CHF₂ oder CF₃; das ein- bis fünffach durch Fluor, Chlor und/oder Brom substituierte Ethyl, wie CH₂CF₃, CF₂CF₃, CF₂CCl₃, CF₂CHCl₂, CF₂CHF₂, CF₂CFCl₂, CF₂CHBr₂, CF₂CHCIF, CF₂CHBrF oder CCIFCHCIF; das ein- bis siebenfach durch Fluor, Chlor und/oder Brom substituierte Propyl oder Isopropyl, wie CH₂CHBrCH₂Br, CF₂CHFCF₃, CH₂CF₂CF₃ oder CH(CF₃)₂; und das ein- bis neunfach durch Fluor, Chlor und/oder Brom substituierte Butyl oder eines seiner Isomeren, wie CF(CF₃)CHFCF3 oder CH₂(CF₂)₂CF₃.

Eine bevorzugte Untergruppe im Umfang der Formel (I) bilden Verbindungen, worin R für verzweigtes C₁-C₁₂ Alkyl, vorzugsweise C₄-C₉ Alkyl steht.
Besonderen Vorzug geniessen Verbindungen der Formel ( I ), worin der Substituent R unsubstituiert ist.
Bevorzugt im Umfang der vorliegenden Erfindung sind Verbindungen, worin X und Y unabhängig voneinander eine geradkettige oder verzweigte Alkylenbrücke mit 1 bis 3 Kohlenstoffatomen bilden, welche unsubstituiert ist. Besonderen Vorzug hat Ethylen.

Hervorzuheben sind Verbindungen der Formel ( I ) worin R1 und R2 für unverzweigtes C₁-C₆ Alkyl, vorzugsweise Methyl oder Ethyl stehen.
Ein besonders bevorzugter Vertreter der Verbindungen der Formel ( I ) ist 2-Propylpentansäure-bis-(2-methoxy-ethyl)-amid, welches folgende Strukturformel besitzt: 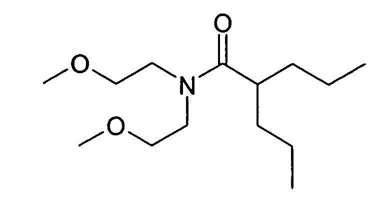 Einen weiteren Gegenstand der vorliegenden Erfindung bildet das Verfahren zur Herstellung der erfindungsgemässen Verbindungen , welches zu Verbindungen der Formel (I) in freier Form oder in Salzform führt. Dieses Verfahren ist dadurch gekennzeichnet, dass man eine Verbindung der Formel 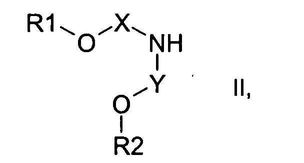 die bekannt ist oder in Analogie zu entsprechenden bekannten Verbindungen hergestellt werden kann und worin R₁, R₂, X, Y die für die Formel (I) angegebenen Bedeutungen haben, mit einer Verbindung der Formel 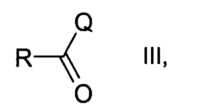 die ebenfalls bekannt ist oder in Analogie zu entsprechenden bekannten Verbindungen hergestellt werden kann und worin R die für die Formel (I) angegebenen Bedeutungen hat und Q eine Abgangsgruppe darstellt, gegebenenfalls in Gegenwart eines basischen Katalysators, umsetzt, und jeweils, wenn erwünscht, eine verfahrensgemäss oder auf andere Weise erhältliche Verbindung der Formel ( I ), jeweils in freier Form oder in Salzform, in eine andere Verbindung der Formel ( I ) überführt, ein verfahrensgemäss erhältliches Gemisch von Isomeren nach an sich bekannten Methoden auftrennt und das gewünschte Isomere isoliert und/oder eine verfahrensgemäss erhältliche freie Verbindung der Formel ( I ) in ein Salz oder ein verfahrensgemäss erhältliches Salz einer Verbindung der Formel ( I ) in die freie Verbindung der Formel ( I ) oder in ein anderes Salz überführt.

Für vor- und nachstehend aufgeführte Ausgangsmaterialien gilt im Hinblick auf deren Salze das vorstehend für Salze von Verbindungen I Gesagte in analoger Weise.
Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid.
Bevorzugte Abgangsgruppen Q sind Halogene, Tosylate, Mesylate und Triflate, besonders bevorzugt Halogene, insbesondere Chlor.
Geeignete Basen zur Erleichterung der Umsetzung sind z. B. Alkalimetall- oder Erdalkalimetallhydroxide, -hydride, -amide, -alkanolate, -acetate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natriumhydroxid, -hydrid, -amid, -methanolat, - acetat, -carbonat, Kalium-t.-butanolat, -hydroxid, -carbonat, -hydrid, Lithiumdiisopropylamid, Kalium-bis(trimethylsilyl)-amid, Calciumhydrid, Triethylamin, Diisopropylethylamin, Triethylendiamin, Cyclohexylamin, N-Cyclohexyl-N,N-dimethylamin, N,N-Diethylanilin, Pyridin, 4-(N,N-Dimethylamino)pyridin, Chinuclidin, N-Methylmorpholin, Benzyltrimethylammoniumhydroxid sowie 1,5-Diazabicyclo[5.4.0]undec-5-en (DBU) genannt. Bevorzugt sind Diisopropylethylamin und 4-(N,N-Dimethylamino)pyridin.
Die Umsetzung erfolgt vorteilhaft in einem Temperaturbereich von etwa 0°C bis etwa +100°C, bevorzugt von etwa 10°C bis etwa +40°C.
Die Verbindungen der Formel (I) sind farblose bis hellgelbe, geschmacksneutrale Öle, die relativ leicht flüchtig sind. Kinetische Untersuchungen zeigen, dass sie nach topikaler Applikation nicht in die Haut eindringen. Man kann weder messbare Blutspiegel der Verbindungen der Formel (I) noch denkbarer Abbauprodukte feststellen. Demzufolge handelt es sich bei topikaler Applikation um kein therapeutisches Verfahren am tierischen oder Menschlichen Körper.

Unter Ungeziefer versteht man im Zusammenhang mit der vorliegenden Erfindung, insbesondere Insekten, Milben und Zecken. Eingeschlossen sind Insekten der Ordnungen: *Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.* Besonders ewähnt sei jedoch Ungeziefer, welches Menschen oder Tiere plagt und Krankheitserreger überträgt, wie z. B. Fliegen, wie *Musca domestica, Musca vetustissima, Musca autumnalis, Fannia canicularis, Sarcophaga carnaria, Lucilia cuprina, Hypoderma bovis, Hypoderma lineatum, Chrysomyia chloropyga, Dermatobia hominis, Cochliomyia hominivorax, Gasterophilus intestinalis, Oestrus ovis, Stomoxys calcitrans, Haematobia irritans* und Mücken *(Nematocera),* wie *Culicidae, Simuliidae, Psychodidae,* aber auch blutsaugendes Ungeziefer, wie beispielsweise Flöhe, wie *Ctenocephalides felis und Ctenocephalides canis* (Katzen- und Hundeflöhe), *Xenopsylla cheopis, Pulex irritans, Dermatophilus penetrans,* Läuse, wie *Damalina ovis, Pediculus humanis*, Stechfliegen und Bremsen *(Tabanidae), Haematopota spp.* wie *Haematopota pluvialis, Tabanidea spp.* wie *Tabanus nigrovittatus, Chrysopsinae* spp. wie *Chrysops caecutiens,* Tsetsefliegen, wie Glossiniaarten, beissende Insekten, allen voran Schaben, wie *Blatella germanica, Blatta orientalis, Periplaneta americana,* Milben wie *Dermanyssus gallinae, Sarcoptes scabiei, Psoroptes ovis und Psorergates spp.* und nicht zuletzt Zecken. Letztere gehören zur Ordnung Acarina. Bekannte Vertreter von Zecken sind, z.B. *Boophilus, Amblyomma, Anocentor, Dermacentor, Haemaphysalis, Hyalomma, Ixodes, Rhipicentor, Margaropus, Rhipicephalus, Argas, Otobius* und *Ornithodoros* und ähnliche, die vorzugsweise warmblütige Tiere, einschliesslich Farmtiere, wie Kühe, Schweine, Schafe und Ziegen, Geflügel wie Hühner, Truthühner und Gänse, Felltiere wie Nerze, Füchse, Chinchillas, Kaninchen und ähnliche, sowie Haustiere wie Katzen und Hunde, aber auch Menschen befallen.
Zecken können in harte und weiche Zecken unterteilt werden und sind dadurch charakterisiert, dass sie einen, zwei oder drei Wirtstiere befallen. Sie heften sich an ein passendes Wirtstier an und saugen Blut oder Körperflüssigkeit. Vollgesogene Zecken-Weibchen lassen sich vom Wirtstier fallen und legen grosse Mengen Eier (2000 bis 3000) in eine passende Nische im Boden oder in irgend einem anderen geschützten Ort, wo die Larven schlüpfen.

Diese suchen wiederum ein Wirtstier, um von ihm Blut zu saugen. Larven von Zecken, welche nur ein Wirtstier befallen, häuten sich zweimal und werden dabei zu Nymphen und schliesslich zu adulten Zecken, ohne den einmal gewählten Wirt zu verlassen. Larven von Zecken, welche zwei oder drei Wirtstiere befallen, lassen sich nach dem Blutmahl fallen, häuten sich in der Umgebung und suchen sich einen zweiten oder dritten Wirt als Nymphe oder adulte Zecke, um an ihm zu saugen.
Zecken sind weltweit für die Uebertragung und Weitergabe von vielen menschlichen und tierischen Krankheiten verantwortlich. Die wegen ihres ökonomischen Einflusses wichtigsten Zecken sind Boophilus, Rhipicephalus, Ixodes, Hyalomma, Amblyomma und Dermacentor. Sie sind Träger für bakterielle, virale, rickettsiale und protozoale Krankheiten und verursachen Zecken-Paralyse und Zecken-Toxikose. Selbst eine einzige Zecke kann eine Paralyse dadurch verursachen, dass ihre Speichelflüssigkeit bei der Nahrungsaufnahme in ihr Wirtstier eindringt. Von Zecken ausgelöste Krankheiten werden gewöhnlich durch Zecken übertragen, welche mehrere Wirtstiere befallen. Solche Krankheiten, wie zum Beispiel Babesiosis, Anaplasmosis, Theileriosis, und Herzwasser sind für den Tod oder Schädigung einer grossen Zahl von Haus- und Farmtieren auf der ganzen Welt verantwortlich. In vielen Ländern mit gemässigtem Klima übertragen Ixodid-Zecken das Agens der chronisch schädigenden Lyme-Krankheit von den Wildtieren auf den Menschen. Neben der Krankheitsübertragung sind die Zecken für grosse wirtschaftliche Verluste in der Viehproduktion verantwortlich. Die Verluste sind nicht nur auf den Tod der Wirtstiere, sondern auch auf die Schädigung der Felle, den Wachstumsverlust, die Verringerung der Milchproduktion und den verminderten Wert des Fleisches zurückzuführen. Obwohl die schädigenden Wirkungen des Zeckenbefalls auf Tieren seit Jahren bekannt sind und enorme Fortschritte mit Zeckenkontroll-Programmen erzielt wurden, sind bis heute keine völlig befriedigenden Methoden zur Kontrolle oder Elimination dieser Parasiten gefunden worden, und ausserdem haben Zecken oft eine Resistenz gegenüber chemischen Wirksubstanzen entwickelt.
Der Befall von Flöhen an Haus- und Schosstieren bildet ebenfalls immer noch ein nur unzureichend oder nur mit erheblichem Aufwand zu lösendes Problem für den Tierbesitzer. Wie Zecken, so sind auch Flöhe nicht nur lästig, sondern Krankheitsüberträber, die vor allem in feucht-warmen klimatischen Zonen, z.B. im Mittelmeerraum, im südlichen Teil der USA, etc., verschiedene Pilzerkrankungen von Wirtstier zu Wirtstier und auf den Tierhalter übertragen. Gefährdet sind vor allem Menschen mit einem geschwächten Imunsystem oder Kinder, deren Imunsystem noch nicht voll entaltet ist. Auf Grund seines komplexen Lebenszykluses ist keine der bekannten Methoden zur Bekämpfung von Flöhen rundum zufriedenstellend, insbesondere, weil die meisten bekannten Methoden prinzipiell auf die Bekämpfung der ausgewachsenen Flöhe im Fell ausgerichtet sind und die unterschiedlichen juvenilen Stadien der Flöhe, die nicht nur im Fell des Tieres, sondern auch auf dem Boden, auf Teppichen, auf dem Schlafplatz des Tieres, auf Stühlen, im Garten und all den andern Plätzen, mit denen das befallene Tier in Berührung kommt, existieren, völlig ausser acht lassen. Die Flohbehandlung ist meistens aufwändig und muss über längere Zeiträume fortgesetzt werden, wobei sich der Erfolg meistens erst dann einstellt, wenn man nicht nur das befallene Tier, z.B. den Hund oder die Katze, behandelt, sondern gleichzeitig alle Plätze an denen sich das befallene Tier häufig aufhält.
Ein solch komplexes Vorgehen ist mit den vorliegenden Verbindungen der Formel ( I ) nicht notwendig, denn ein besonderer Vorteil der hier zur Diskussion stehenden Verbindungen der Formel (I) besteht darin, dass sie äusserst wirksam und gleichzeitig sehr untoxisch sind, sowohl für die Zielparasiten, als auch für Warmblüter. Ihre Wirkung beruht nämlich nicht auf der Tötung des Zielparasiten, sondern auf dessen vertreibende Abwehr und zwar noch bevor er einen Wirsorganismus befällt, sticht, beisst oder ihn anderweitig belästigt. Die Anwesenheit der hier diskutierten Verbindungen der Formel (I) scheint die Parasiten dermassen zu stören, dass sie fluchtartig die behandelte Umgebung verlassen, ohne zu beissen oder zu stechen bzw. ein behandeltes Witrtier garnicht erst befallen. Auffallend ist, dass die Wirkung dann einsetzt, wenn der Parasit kurzfristig mit der Aktivsubstanz in Berührung kommt. Nach kurzem Kontakt meidet der Parasit jede weitere Berührung mit der Aktivsubstanz. Ein zusätzlicher Vorteil liegt in der Langzeitwirkung, z. B. im Vergleich zu DEET (N, N-diethyl-m-toluamide), welches zwar sehr wirksam, aber sich ziemlich rasch verflüchtigt und daher bereits nach ca. 2 Stunden neu aufgetragen werden muss und sich daher zur Dauerbehandlung von Tieren nicht eignet. Die Anwendung der vorliegenden Wirksubstanzen ist auch deshalb angenehm, weil sie nahezu geruchlos sind.
Obwohl man die vorliegenden Wirksubstanzen selbstverständlich zur Erzielung einer noch besseren oder noch länger anhaltenden Wirkung mit anderern Substanzen gleicher Wirkungsrichtung oder mit Parasitiziden oder anderen wirkungsverbessernden Substanzen mischen und zur Anwendung bringen kann, ist dies im Gegensatz zu vielen Verbindungen des Standes der Technik völlig unnötig, da sie alle vorteilhaften Eigenschaften bereits in sich vereinigen.
Sofern nicht nur ein Fernhalten des Parasiten angestrebt wird, sondern dessen Abtötung erreicht werden soll, kann man dies selbstverständlich durch Zusatz von geeigneten Insektiziden und/oder Akariziden erreichen. Bei der praktischen Anwendung ist dies jedoch in den meisten Fällen unnötig.
Die erfindungsgemässen Substanzen werden zweckmässigerweise in Form verdünnter Lösungen auf das Fell eines Tieres oder of die menschliche Haut aufgetragen, darüber hinaus kann man sie aber auch in andere Applikationsformen überführen und in Form von Pasten, Sprays, eingearbeitet in Halsbänder oder Anhänger einsetzen. Diese letztgenannten Anwendungsformen sind dann von besonderem Vorteil, wenn man eine Langzeitwirkung anstrebt, weil Pasten, Halsbänder und Anhänger sich so konzipieren lassen, dass sie relativ grosse Mengen an Wirkstoff aufnehmen und diesen über längere Zeiträume freistzen. Dem Fachmann sind derartige "Slowrelease"-Formulierungen hinlänglich bekannt.

Für die Herstellung der Halsbänder können bekannterweise Polyvinylharze, Polyurethane, Polyacrylate, Epoxyharze, Cellulose, Cellulosederivate, Polyamide und Polyester verwendet werden, die mit den obengenannten Wirkstoffen ausreichend verträglich sind. Die Polymeren sollten eine ausreichende Festigkeit und Biegsamkeit haben, um beim Formen in ein Band nicht zu reissen oder brüchig zu werden. Sie müssen von ausreichender Haltbarkeit sein, um gegen normale Abnutzung beständig zu sein. Ausserdem müssen die Polymeren eine ausreichende Wanderung der Wirkstoffe an die Oberfläche des Formkörpers zulassen. Diese Eigenschaften werden insbesondere von festen Polyvinylharzen erfüllt, d. h. von Polymerisaten, die durch Polymerisaten einer Vinyldoppelbindung gebildet werden. Typische Vinylharze sind beispielsweise Polyvinylhalogenide, wie Polyvinylchlorid, Polyvinylchlorid-Vinylacetat und Polyvinylfluorid; Polyacrylat- und Polymethacrylatester, wie Polymethylacrylat und Polymethylmethacrylat; und Polyvinylbenzole, wie Polystyrol und Polyvinyltoluol.

Für die Herstellung der Halsbänder auf der Basis Polyvinylharz sind solche Weichmacher geeignet, die normalerweise zum Weichmachen von festen Vinylharzen verwendet werden. Der zu verwendende Weichmacher hängt von dem Harz und seiner Verträglichkeit mit dem Weichmacher ab. Geeignete Weichmacher sind beispielsweise Ester von Phosphorsäure, wie Tricresylphosphat, Ester von Phthalsäure, wie Dimethylphthalat und Dioctylphthalat, und Ester von Adipinsäure, wie Diisobutyladipat. Es können auch andere Ester, wie die Ester von Azelainsäure, Maleinsäure, Ricinolsäure, Myristinsäure, Palmitinsäure, Oelsäure, Sebacinsäure, Stearinsäure und Trimellithsäure, sowie komplexe lineare Polyester, polymere Weichmacher und epoxydierte Sojabohnenöle verwendet werden. Die Menge des Weichmachers beträgt etwa 10 bis 50 Gew.-%, vorzugsweise etwa 20 bis 45 Gew.-% der gesamten Zusammensetzung.

In den Halsbändern können noch weitere Bestandteile, wie Stabilisierungsmittel, Spreitmittel, Schmiermittel, Füllstoffe und Färbematerialien, enthalten sein, ohne dass dadurch die grundlegenden Eigenschaften der Zusammensetzung verändert werden. Geeignete Stabilisierungsmittel sind Antioxydationsmittel und Mittel, die die Bänder vor ultravioletter Strahlung und unerwünschtem Abbau während der Bearbeitung, wie Strangpressen schützen. Einige Stabilisierungsmittel, wie epoxydierte Sojabohnenöle, dienen ausserdem als sekundäre Weichmacher. Als Schmiermittel können beispielsweise Stearate, Stearinsäure und Polyethylene mit niedrigem Molekulargewicht verwendet werden. Diese Bestandteile können in einer Konzentration bis zu etwa 5 Gew.-% der gesamten Zusammensetzung verwendet werden.

Bei der Herstellung von Halsbändern auf Vinylbasis werden die verschiedenen Bestandteile nach bekannten Mischverfahren trocken gemischt und nach bekannten Extrusionsverfahren hergestellt.

Die Wahl des Verarbeitungsverfahrens zur Herstellung der erfindungsgemässen Halsbänder richtet sich technisch grundsätzlich nach den rheologischen Eigenschaften des Bandmaterials und der Form des gewünschten Bandes. Die Verarbeitungsverfahren können nach der Verarbeitungstechnologie oder nach der Art der Formgebung eingestellt werden. Bei der Verfahrenstechnologie kann man die Verfahren nach den bei ihnen durchlaufenden rheologischen Zuständen unterteilen. Danach kommen für viskose Bandmaterialien Giessen, Pressen, Spritzen und Auftragen und für elastoviskose Polymere Spritzgiessen, Strangpressen (Extrudieren), Kalandrieren, Walzen und gegebenenfalls Kanten in Frage. Nach Art der Formgebung eingeteilt, lassen sich die erfindungsgemässen Formkörper durch Giessen, Tauchen, Pressen, Spritzgiessen, Extrudieren, Kalandrieren, Prägen, Biegen, Tiefziehen etc. herstellen.

Diese Verarbeitungsverfahren sind bekannt und bedürfen keiner näheren Erklärung. Im Prinzip gelten für Polymere wie Polyamide und Polyester die Erläuterungen, die oben beispielhaft für Polyvinylharze gemacht wurden.

Weitere Trägermaterialien für die erfindungsgemässen Halsbänder sind Polyurethane. Diese werden in an sich bekannter Weise durch Umsetzung von Polyisocyanaten mit höhermolekularen, mindestens zwei gegenüber Isocyanaten reaktionsfähigen Gruppen aufweisenden Verbindungen sowie gegebenenfalls niedermolekularen Kettenverlängerungsmitteln und/oder monofunktionellen Kettenabbrechern hergestellt.

Die Wirkstoffe liegen in den Trägerpolymeren in Konzentrationen von 0,1-30 Gew.-% vor. Bevorzugt sind Konzentrationen von 10-20 Gew.-%. Besonders bevorzugt ist eine WirkstoffKonzentration um ca. 20 Gewichtsprozent.

Die vorliegenden Wirksubstanzen werden vorzugsweise verdünnt eingesetzt. Üblicherweise werden sie mit geigneten Formulierungshilfsstoffen in die endgültige Anwendungsform gebracht, wobei letztere zwischen 0.1 und 95 Gew.-%, vorzugsweise 0.5 bis 90 Gew.-% des Wirkstoffes enthalten. Einfache alkoholische Lösungen in niederen Alkanolen, wie Ethanol, Propanol oder Isopropanol lassen sich mit grossem Erfolg einsetzen, ohne dass weiter Hilfsstoffe nötig wären. Solche einfachen Lösungen sind im Rahmen der vorliegenden Erindung besonders bevorzugt.
Da die Wirkstoffe in vielen Fällen an Warmblüter appliziert werden und naturgemäss in Kontakt mit der Haut geraten, eignen sich als Formulierungshilfsstoffe auch die aus der Kosmetik bekannten Hilfsstoffe und Verabreichungsformen. Man kann sie in Form von Lösungen, Emulsionen, Salben, Cremes, Pasten, Pulvern, Sprays, etc. verabreichen. Für die Verabreichung an Nutz- oder Schoss- und Stalltiere, wie Kühe, Pferde, Esel, Kamele, Hunde, Katzen, Hühner, Schafe, Ziegen etc. eignen sich besonders sogenannte 'Pour-on' oder Spot-on' Formulierungen, diese Flüssig- oder Halbflüssigformulierungen haben den Vorteil, dass man sie nur auf eine kleine Fläche des Fells oder des Federkleides aufzutragen braucht und sie sich dank dem Anteil an spreitenden Ölen oder anderer spreitender Zusätzen, ohne weiteres Zutun, auf das ganze Fell bzw. Federkleid selbständig verteilen und flächendeckend zur Wirkung gelangen.
Selbstverständlich lassen sich auch unbelebte Materialien, z.B. Bekleidung oder Hunde- und Katzenkörbchen, Stallungen, Teppiche, Vorhänge, Wohnungen, Wintergärten, etc. mit besagten Formulierungen behandeln und so vor Parasitenbefall schützen.
Zur Bekämpfung von Schaben können deren Aufenthaltsorte, meistens Ritzen in Wänden, Möbeln etc., eingesprüht oder eingepudert werden. Da Schaben äusserst vital sind und ein entgültiges Vertreiben kaum erreicht werden kann, empfiehlt es sichbeim Einsatz der vorliegenden Wirkstoffe zusätzlich gegen Schaben wirksame Insektizide einzusetzen.
Für die Anwendung am Menschen kann man eine wohlriechende Essenzen, z.B. ein Parfum, zusetzen um die Anwendung attraktiver zu gestalten.
Die folgenden Beispiel für die Zubereitungen und die Verwendung der erfindungsgemässen Wirkstoffe dienen der Erläuterung der Erfindung, ohne sie einzuschränken. Insbesondere setzen sich bevorzugte Formulierungen folgendermassen zusammen:

### Formulierunasbeispiel 1

Ein ungeziefer-abwehrendes Mittel in Form einer Lotion zur Anwendung auf der Haut wird hergestellt durch Vermischen von 30 Teilen eines der erfindungsgemässen Wirkstoffe aus Tabelle 1, 1,5 Teilen Parfum und 68,5 Teilen Isopropanol, wobei letzteres durch Ethanol ersetzt werden kann.

### Formulierungsbeispiel 2

Ein ungeziefer-abwehrendes Mittel in Form eines Aerosols zum Aufsprühen auf das Fell eines Schosstieres wird hergestellt, indem man 50% Wirkstofflösung, bestehend aus 30 Teilen eines der erfindungsgemässen Wirkstoffe aus Tabelle 1, 1,5 Teilen Parfum und 68,5 Teilen Isopropanol, mit 50% Frigen 11/12 (einem halogenierten Kohlenwasserstoff) als Treibgas in einer Sprühdose formuliert.

### Formulierungsbeispiel 3

Ein ungeziefer-abwehrendes Mittel in Form eines Aerosols zum Aufsprühen auf die Haut wird hergestellt, indem man 40% Wirkstofflösung, bestehend aus 20 Teilen eines der erfindungsgemässen Wirkstoffe, 1 Teil Parfüm, 79 Teilen Isopropanol, mit 60% Propan/Butan (im Verhältnis 15:85) als Treibgas in einer Sprühdose formuliert.

### Herstellungsbeispiel: Herstellung von 2-Propyl-pentansäure-bis-(2-methoxy-ethyl)-amid ( Vernindung Nr. 1.31 in der nachfolgenden Tabelle 1) mit der nachstehenden Formel

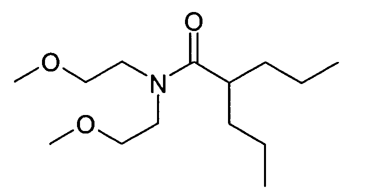

Zu einer Lösung von 75g (0.56 Mol) Bis-(2-methoxy-ethyl)-amin und 50,6g (0.50 Mol)Triethyl amin in 700ml Methylenchlorid lässt man bei 10 °C eine Lösung von 162,5g (0.50Mol) 2-Propyl-pentanoyl chlorid in 300ml Methylenchlorid zutropfen. Man rührt das Reaktionsgemisch ca 12h bei Raumtemperatur und wäscht danach die Reaktionslösung mit je 250ml H₂O, 1 N NaOH, 1 N HCl und wässriger Kochsalzlösung. Anschliessend wird die organische Phase abgetrennt und eingedampft und der Rückstand im Hochvakuum (0,1 Torr) über eine Vigreux Kolonne destilliert (Siedtemperatur: 108-112 °C). Man erhält 121g (93%) eines farb- und geruchlosen Gels.
Wie bereits ausgeführt handelt es sich bei den Verbindungen der Formel ( I ) um farblose bis hellgelbe, geschmacksneutrale Öle, die relativ leicht flüchtig sind.

Die nachfolgenden Tabelle 1 zeigt bevorzugte Vertreter von Verbindungen der Forme (I).

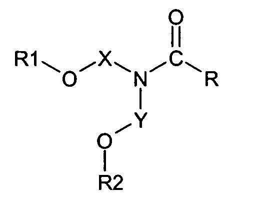

**Tablle 1:**

| Verbindungen der Formel ( I ) | | | | | |
|---|---|---|---|---|---|
| **No.** | **R** | **X** | **Y** | **R1** | **R2** |
| | | | | | |
| 1.01 | CH₃ | CH₂ | CH₂ | CH₃ | CH₃ |
| 1.02 | CH₃ | CH₂ | CH₂ | C₂H₅ | CH₃ |
| 1.03 | CH₃ | CH₂ | CH₂ | C₂H₅ | C₂H₅ |
| 1.04 | CH₃ | (CH₂)₂ | CH₂ | C₂H₅ | C₂H₅ |
| 1.05 | CH₃ | (CH₂)₂ | (CH₂)₂ | C₂H₅ | C₂H₅ |
| 1.06 | C₂H₅ | CH₂ | CH₂ | CH₃ | CH₃ |
| 1.07 | C₂H₅ | CH₂ | CH₂ | C₂H₅ | CH₃ |
| 1.08 | C₂H₅ | CH₂ | CH₂ | C₂H₅ | C₂H₅ |
| 1.09 | C₂H₅ | (CH₂)₂ | CH₂ | C₂H₅ | C₂H₅ |
| 1.10 | C₂H₅ | (CH₂)₂ | (CH₂)₂ | C₂H₅ | C₂H₅ |
| 1.11 | C₃H₇-n | CH₂ | CH₂ | CH₃ | CH₃ |
| 1.12 | C₃H₇-n | CH₂ | CH₂ | C₂H₅ | CH₃ |
| 1.13 | C₃H₇-n | CH₂ | CH₂ | C₂H₅ | C₂H₅ |
| 1.14 | C₃H₇-n | (CH₂)₂ | CH₂ | C₂H₅ | C₂H₅ |
| 1.15 | C₃H₇-n | (CH₂)₂ | (CH₂)₂ | C₂H₅ | C₂H₅ |
| 1.16 | C₃H₇-i | CH₂ | CH₂ | CH₃ | CH₃ |
| 1.17 | C₃H₇-i | CH₂ | CH₂ | C₂H₅ | CH₃ |
| 1.18 | C₃H₇-i | CH₂ | CH₂ | C₂H₅ | C₂H₅ |
| 1.19 | C₃H₇-i | (CH₂)₂ | CH₂ | C₂H₅ | C₂H₅ |
| 1.20 | C₃H₇-i | (CH₂)₂ | (CH₂)₂ | C₂H₅ | C₂H₅ |
| 1.21 | CH(C₂H₅)₂ | CH₂ | CH₂ | CH₃ | CH₃ |
| 1.22 | CH(C₂H₅)₂ | CH₂ | CH₂ | C₂H₅ | CH₃ |
| 1.23 | CH(C₂H₅)₂ | CH₂ | CH₂ | C₂H₅ | C₂H₅ |
| 1.24 | CH(C₂H₅)₂ | (CH₂)₂ | CH₂ | C₂H₅ | C₂H₅ |
| 1.25 | CH(C₂H₅)₂ | (CH₂)₂ | (CH₂)₂ | C₂H₅ | C₂H₅ |
| 1.26 | CH(C₂H₅)(C₃H₇-n) | CH₂ | CH₂ | CH₃ | CH₃ |
| 1.27 | CH(C₂H₅)(C₃H₇-n) | CH₂ | CH₂ | C₂H₅ | CH₃ |
| 1.28 | CH(C₂H₅)(C₃H₇-n) | CH₂ | CH₂ | C₂H₅ | C₂H₅ |
| 1.29 | CH(C₂H₅)(C₃H₇-n) | (CH₂)₂ | CH₂ | C₂H₅ | C₂H₅ |
| 1.30 | CH(C₂H₅)(C₃H₇-n) | (CH₂)₂ | (CH₂)₂ | C₂H₅ | C₂H₅ |
| 1.31 | CH(C₃H₇-n)₂ | CH₂ | CH₂ | CH₃ | CH₃ |
| 1.32 | CH(C₃H₇-n)₂ | CH₂ | CH₂ | C₂H₅ | CH₃ |
| 1.33 | CH(C₃H₇-n)₂ | CH₂ | CH₂ | C₂H₅ | C₂H₅ |
| 1.34 | CH(C₃H₇-n)₂ | (CH₂)₂ | CH₂ | C₂H₅ | C₂H₅ |
| 1.35 | CH(C₃H₇-n)₂ | (CH₂)₂ | (CH₂)₂ | C₂H₅ | C₂H₅ |
| 1.36 | CH(C₃H₇-n)(C₄H₉-n) | CH₂ | CH₂ | CH₃ | CH₃ |
| 1.37 | CH(C₃H₇-n)(C₄H₉-n) | CH₂ | CH₂ | C₂H₅ | CH₃ |
| 1.38 | CH(C₃H₇-n)(C₄H₉-n) | CH₂ | CH₂ | C₂H₅ | C₂H₅ |
| 1.39 | CH(C₃H₇-n)(C₄H₉-n) | (CH₂)₂ | CH₂ | C₂H₅ | C₂H₅ |
| 1.40 | CH(C₃H₇-n)(C₄H₉-n) | (CH₂)₂ | (CH₂)₂ | C₂H₅ | C₂H₅ |
| 1.41 | CH(C₄H₉-n)₂ | CH₂ | CH₂ | CH₃ | CH₃ |
| 1.42 | CH(C₄H₉-n)₂ | CH₂ | CH₂ | C₂H₅ | CH₃ |
| 1.43 | CH(C₄H₉-n)₂ | CH₂ | CH₂ | C₂H₅ | C₂H₅ |
| 1.44 | CH(C₄H₉-n)₂ | (CH₂)₂ | CH₂ | C₂H₅ | C₂H₅ |
| 1.45 | CH(C₄H₉-n)₂ | (CH₂)₂ | (CH₂)₂ | C₂H₅ | C₂H₅ |
| 1.46 | CH(C₅H₁₁-n)₂ | CH₂ | CH₂ | CH₃ | CH₃ |
| 1.47 | CH(C₅H₁₁-n)₂ | CH₂ | CH₂ | C₂H₅ | CH₃ |
| 1.48 | CH(C₅H₁₁-n)₂ | CH₂ | CH₂ | C₂H₅ | C₂H₅ |
| 1.49 | CH(C₅H₁₁-n)₂ | (CH₂)₂ | CH₂ | C₂H₅ | C₂H₅ |
| 1.50 | CH(C₅H₁₁-n)₂ | (CH₂)₂ | (CH₂)₂ | C₂H₅ | C₂H₅ |
| 1.51 | CH(C₆H₁₃-n)₂ | (CH₂)₂ | (CH₂)₂ | C₂H₅ | C₂H₅ |
| 1.52 | CH(C₆H₁₃-n)₂ | CH₂ | CH₂ | CH₃ | CH₃ |
| 1.53 | CH(C₆H₁₃-n)₂ | CH₂ | CH₂ | C₂H₅ | CH₃ |
| 1.54 | CH(C₆H₁₃-n)₂ | CH₂ | CH₂ | C₂H₅ | C₂H₅ |
| 1.55 | CH(C₆H₁₃-n)₂ | (CH₂)₂ | CH₂ | C₂H₅ | C₂H₅ |

### Bioloctische Beispiele:

### Arena-Test - Methode zum Testen von Ungeziefer-abweisenden Substanzen

Die Methode wird in Titerplatten mit 6 Vertiefungen mit einem Querschnitt von jeweils 5 cm und unter Zuhilfenahme eines computer-unterstützten Viedeoaufnahmesystems durchgeführt. Jede Vertiefung der Titerplatte wird mit einem kreisrunden Filterpapier oder einem anderen geeigneten Trägermaterial ausgelegt. Die zu testende Substanz der Formel (I) wird in Methanol, Acetonitril oder einem anderen geeigneten Lösungsmittel, bei schwerlöslichen Substanzen unter Einwirkung von Ultraschall und Erwärmung, gelöst. Die gelöste Testsubstanz wird in einer Menge von 1 bis 100 µg/cm² auf einen einen Quadranten oder auf einen kreisrunden Bereich mit ca. 2.4 cm² Radius im Zentrum des Filterpapiers aufgetragen. 4 der 6 Vertiefungen werden mit unterschiedlichen Testsubstanzen oder mit derselben Testsubstanz in unterschiedlichen Verdünnungen (z.B. 1, 3,2, 5, 10 und 20 µg/cm²) bestückt. Die 5. Vertiefung wird mit DEET (N,N-diethyl-m-toluamide) als Standardsubstanz behandelt. Die 6. Vertiefung wird mit dem reinen Lösungsmittel bestückt und dient als Kontrolle. Auf jedes Filterpapier werden 60 bis 100 Larven oder 25 bis 50 Nymphen oder 10 bis 25 Adulte des zu testenden Parasiten, z.B. Zecken, gegeben und das System mit einer Glasscheibe abgedeckt und unter eine Videokamera positioniert.
Die Videokamera nimmt in Abständen von 5 Sekunden Einzelbilder aller 6 Vertiefungen auf. Man betrachtet zur qualitativen Auswertung diese Bilder im Zeitraffer wie einen fortlaufenden Film, wobei man die Bewegungen der Parasiten auf dem Filterpapier optisch verfolgt und mit den Bewegungen in der Kontrollvertiefung Nr.6 oder mit dem Standard in der 5. Vertiefung vergleicht. Dabei beobachtet man qualitativ, ob die Testparasiten sich gleichmässig auf der gesamten Filterpapieroberfläche bewegen und die Testsubstanz ignorieren oder ob und über welchen Zeitraum sie die behandelte Zone meiden und welchen Einfluss die Verdünnung der Testsubstanz auf das Verhalten des Testparasiten hat. Auf diese Weise ermittelt man neutrale und abweisende Substanzen. Gleichzeitig wird dabei die Wirkungsdauer der Testsubstanz bestimmt und mit der des Standards verglichen. Projiziert man alle Bilder für jede einzelne Vertiefung übereinander, so ergeben sich unterschiedliche Schwärzungsbereiche. Diese repräsentieren die Aufenthaltshäufigkeit der Parasiten an bestimmten Orten. Diese Aufenthaltshäufigkeit wird im Vergleich mit der Kontrolle und des Standards nach der Methode von Willcoxon statistisch und damit quantitativ ausgewertet. Verbindungen aus Tabelle 1, wie z.B. Nr. 1.11 bis 1.31, 1.45, 1.47 und 1.55 zeigen eine ausgezeichenete Wirkung. Als besonder aktiv erweist sich die Verbindung Nr. 1.31

### Arena-Test: in vitro gegen Amblyomma hebraeum oder variegatum (Nymphen)

Der Test wird wie oben beschrieben durchgeführt, wobei pro Vertiefung ca. 25 bis 50 Nymphen gegeben werden. Man trägt 10 mg gelöste Testsubstanz auf eine Fläche von 2.4 cm² Radius auf. Die Auswertung der Videoaufnahmen zeigt, dass die Verbindungen der Formel ( I ) eine ausgeprägte abweisende Wirkung gegen Amblyomma Nymphen entfalten, die wesentlich länger anhält, als diejenige von DEET. Eine besonders ausgeprägte Langzeitwirkung zeigen z.B. die Verbindung Nr. 1.31 sogar bis zu einer Verdünnung auf 3.2 µg/cm^{2.}.

### Arena-Test: in vitro gegen Boophilus microplus Biarra (Larven)

Der Test wird wie oben beschrieben durchgeführt, wobei pro Vertiefung ca. 60 bis 100 Larven gegeben werden. Man trägt 10 mg gelöste Testsubstanz auf eine Fläche von 2.4 cm² Radius auf. Die Auswertung der Videoaufnahmen zeigt, dass die Verbindungen der Formel ( I) eine ausgeprägte abweisende Wirkung gegen Bophilus Larven entfalten, die wesentlich länger anhält, als diejenige von DEET. Eine besonders ausgeprägte Langzeitwirkung zeigen z.B. die Verbindung Nr. 1,31 sogar bis zu einer Verdünnung auf 3.2 µg/cm^{2.}.

### Arena-Test: in vitro gegen Rhipicephalus sanquineus (Nymphen)

Analog zu Beispiel B wird ein Test mit ca. 40 bis 50 Nymphen durchgeführt. Die Auswertung der Videoaufnahmen zeigt, dass die erfindungsgemässen Verbindungen gute abweisende Wirkung zeigen. Insbesondere zeichnen sich die Verbindungen durch eine fast vollständige abweisende Wirkung aus, die wesentlich länger anhält, als diejenige von DEET. Eine besonders ausgeprägte Langzeitwirkung zeigen z.B. die Verbindung Nr. 1,31 sogar bis zu einer Verdünnung auf 3.2 µg/cm^{2.}.

In analogen Versuchsanordnungen werden die gleichen Testsubstanzen hinsichtlich ihrer anweisenden Wirkung an verschiedenen Fliegenspezies, wie z.B. Musca domestica, geprüft. Dabei zeigt es sich, dass die bereits oben genannten Substanzen auch bei diesen diesen Modellversuchen eine stark abweisende Wirkung entfalten.

### In-vivo Vergleich an Hunden hinsichtlich der anti-Zeckenwirkung von 2-Propyl-pentansäurebis-(2-methoxy-ethyl)-amid gemäss vorliegender Erfindung mit DEED (in Form Parapic Dog®) nach Spray-Applikation

Bei dem nachfolgenden Versuch werden folgende Wirkstofformulierungen eingesetzt:
Zusammensetzung des Parapic Anti-Zecken-Sprays:
   Activsubstanz: 15% Diethyltoluamide (DEET); 15% Ethylbutylacetylaminopropionat (EBAAP), Isopropanol, Methacrylicsäure Copolymer, Carbamide, Fragrance (Parfum).
Zusammensetzung des 2-Propyl-pentansäure-bis-(2-methoxy-ethyl)-amid-Spays:
   Activsubstanz: 2-Propyl-pentansäure-bis-(2-methoxy-ethyl)-amid 4.5%; Pluronic F6® 2.0%; Wasser 10.0%, Isopropanol ad 100.0%.

Pluronic® ist eine nichtionische oberflächenaktive Substant (surfactant) bestehend aus den Blockcopolymeren von Propylenoxid und Ethylenoxid.

Ziel des Versuchs ist es, unter natürlichen Bedingungen das kommerziell erhältliche Parapic Dog®, welches gemäss Packungsangabe eine 80%ige anti-Zeckenwirkung über einen Zeitraum von 48 Stunden verspricht, mit einem typischen Vertreter einer Verbindung der Formel ( I ), nämlich 2-Propyl-pentansäure-bis-(2-methoxy-ethyl)-amid vergleichend zu prüfen. Das in Parapic Dog® enthaltene DEED ist der derzeit am meisten gegen Zecken bei Hunden und Katzen aber auch Menschen verwendete Wirkstoff.

Es werden 12 gleichaltrige Hunde der Rasse Beagle in Gruppen in je 4 Hunden eingeteilt. Zur Unterscheidung erhält jeder Hund eine nummerierte Marke. Gruppe 1 wird mit einem 4,5%igen 2-Propyl-pentansäure-bis-(2-methoxy-ethyl)-amid-Spay (3645mg a.i./m²) behandelt. Gruppe 2 wird mit Parapic Dog®-Spray (20% DEED / 3645mg a.i./m²) behandelt. Gruppe 3 bleibt unbehandelt und dient als Kontrolle.
Alle zwölf Hunde werden an 3 aufeinander folgenden Tagen zu einem mit Zecken der Gattung *Ixodes ricinus* infizierten Waldstück gebracht und dort frei laufengelassen. Sie dürfen sich dort 2 Stunden frei bewegen. Die Auswertung erfolgt unmittelbar nach Rückkehr aus dem Wald. Die zweistündigen Spaziergänge werden an den 2 nachfolgenden Tagen wiederholt, ohne dass die Hunde erneut behandelt werden und die Auswertung wieder unmittelbar nach Rückkehr aus dem Wald vorgenommen. Zu diesem Zweck wird das Fell und die Haut jedes Tieres sorgfältig nach anhaftenden Zecken abgesucht. Die Zecken werden gezählt und mit der Anzahl von Zecken der Kontrollgruppe verglichen. Bei keinem der Hunde werden Hautirritationen oder sonstige unerwünschte Nebeneffekte festgestellt.

Der Versuch führt zu folgenden Resultaten:

| | Anti-Zeckenwirkung | | |
|---|---|---|---|
| Substanz | Nach 1 Tag | Nach 2 Tagen | Nach 3 Tagen |
| Parapic | 98% | 70% | -- |
| 2-Propylpentansäure-bis-(2-methoxy-ethyl)-amid | 100% | 98% | 79% |

Der Vergleich zeigt, dass die erfindungsgemässe Substanz 2-Propyl-pentansäure-bis-(2-methoxy-ethyl)-amid dem derzeit am häufigsten verwendeten Deed signifikant überlegen ist und während 3 aufeiander folgenden Tagen zu sehr guten Resultaten führt, während die Aktivität von Deed nach 2 Tagen aprupt abfällt.

Die erfindungsgemässen Verbindungen der Formel (I) sind demzufolge für die Herstellung eines Produktes geeignet, das nach einmaliger Applikation ein ganzes Wochenende vor Zecken schützt.

## Patentansprüche

1. Verbindung der Formel ( I ) 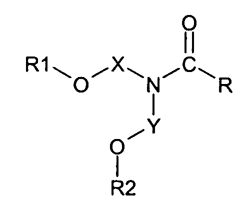 worin
R für unverzweigtes oder verzweigtes C₁-C₁₅ Alkyl, vorzugsweise verzweigtes C₁-C₉ Alkyl steht, welches unsubstituiert oder durch Halogen, Cyano oder Nitro substituiert ist; R1 und
R2 für unverzweigtes oder verzweigtes C₁-C₁₂ Alkyl, vorzugsweise C₁-C₆ Alkyl stehen, welches unsubstituiert oder durch Halogen, Cyano oder Nitro substituiert ist; und X und Y unabhängig voneinander eine geradkettige oder verzweigte Alkylenbrücke mit 1 bis 20 Kohlenstoffatomen, vorzugsweise eine Alkylenbrücke mit 1 bis 3 Kohlenstoffatomen bilden, welche unsubstituiert oder durch Halogen, Cyano oder Nitro substituiert ist.

2. Verbindung der Formel ( I ) nach Anspruch 1, worin R für CH(C₁-C₄ alkyl)₂ steht wobei die beiden verschieden oder vorzugsweise gleich lang und uverzweigt oder vorzugsweise unverzweigt sind.

3. Verbindung der Formel (I) nach einem der Ansprüche 1 oder 2, worin worin R für CH(C₃H₇-n)₂steht

4. Verbindung der Formel ( I ) nach einem der Ansprüche 1 bis 3, worin X und Y unabhängig voneinander für Methylen oder Ethylen stehen.

5. Verbindung der Formel ( I ) nach einem der Ansprüche 1 bis 4, worin R1 und R2 unabhängig voneinander für Methylen oder Ethylen stehen.

6. Die Verbindung 2-Propyl-pentansäure-bis-(2-methoxy-ethyl)-amid nach Anspruch 1.

7. Verfahren zur Herstellung einer Verbindung der Formel ( I ), **dadurch gekennzeichnet, dass** man man eine Verbindung der Formel 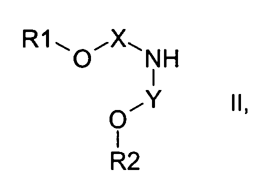 worin R₁, R₂, X, Y die für die Formel ( I ) angegebenen Bedeutungen haben, mit einer Verbindung der Formel 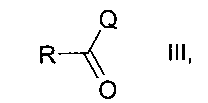 worin R die für die Formel ( I ) angegebenen Bedeutungen hat und Q eine Abgangsgruppe darstellt, gegebenenfalls in Gegenwart eines basischen Katalysators, umsetzt, und eine in freier Form erhaltene Verbindung der Formel ( I ) in eine Salzform überführt oder eine in Salzform erhaltene Verbindung der Formel ( I ) in ihre freie Form überführt und ein erhaltenes Isomerengemisch auftrennt.

8. Verfahren nach Anspruch 7, **dadurch gekennzeichnet, dass** man die Reakanten ohne Zusatz eines Lösungs- oder Verdünnungsmittels miteinander umgesetzt.

9. Verfahren nach Anspruch 7, **dadurch gekennzeichnet, dass** man die Reaktanten in Gegenwart eines geeigneten inerten Lösungsmittels oder Lösungsmittelgemisches und bei Temperaturen von etwa 0-100°C umsetzt.

10. Verfahren nach einem der Ansprüche 7 bis 9, **dadurch gekennzeichnet, dass** man die Reaktion in Gegenwart einer geeigneten Base durchführt.

11. Verfahren nach einem der Ansprüche 7 bis 9, **dadurch gekennzeichnet, dass** man eine Verbindung der Formel (III) einsetzt, worin die Abgangsgruppe Q für Halogen, Tosylate, Mesylate und Triflate, und besonders bevorzugt für ein Halogene steht.

12. Ungeziefer-abweisende Mittel, **dadurch gekennzeichnet, dass** es als Wirkstoff eine Verbindung der Formel ( I ) nach einem der Ansprüche 1-6 und mindestens ein geeignetes Verdünnungsmittel oder einen spreitenden Zusatz enthält.

13. Ungeziefer-abweisende Mittel nach Anspruch 12, **dadurch gekennzeichnet**, das es in Form einer alkohollischen Lösung vorliegt.

14. Ungeziefer-abweisende Mittel nach Anspruch 12, **dadurch gekennzeichnet**, das es in Form einer Pour-on- oder Spot-on-Formulierung vorliegt.

15. Ungeziefer-abweisende Mittel nach Anspruch 12, **dadurch gekennzeichnet**, das es in Form eines Halsbandes oder Anhängers vorliegt.

16. Verwendung einer Verbindung der Formel (I) nach einem der Ansprüche 1-6 zum Fernhalten von Ungeziefer von einem Tier oder einem Menschen oder einem Gegenstand, **dadurch gekennzeichnet, dass** man in einem nicht-therapeutischen Verfahren topikal auf das Tier, den Menschen oder den Gegenstand eine das Ungeziefer abweisende Menge der Verbindung der Formel ( I ) appliziert.

17. Verwendung einer Verbindung der Formel ( I ) nach einem der Ansprüche 1-6 zur Herstellung eines Mittels nach Anspruch 12.

18. Verwendung einer Verbindung der Formel ( I ) nach einem der Ansprüche 1-6 in einem Verfahren zum Abweisen von Ungeziefer von Tieren, Menschen oder Gegenständen.

19. Verfahren zum Fernhalten von Ungeziefer von Orten oder Materialien, an denen sie unerwünscht sind, **dadurch gekennzeichnet, dass** man eine wirksame Menge einer Verbindung der Formel (I) gemäss einem der Ansprüche 1-6 auf den Ort oder das Material appliziert, von dem man das Ungeziefer fernhalten möchte.

20. Verfahren zur Herstellung eines Mittel zum Abweisen von Ungeziefer, **dadurch gekennzeichnet, dass** man eine Verbindung der Formel (I) gemäss einem der Ansprüche 1-6 mit einem spreitenden Zusatz mischt.
